# EUROPEAN PATENT APPLICATION

(11) **EP 4 552 616 A1**
(43) Date of publication of application: **14.05.2025**
(21) Application number: 23208789.0
(22) Date of filing: 09.11.2023
(51) Int. Cl.: A61F 2/46, A61B 17/16, A61B 17/72, A61B 17/92, A61F 2/42

(54) **A SURGICAL BROACHING TOOL**

(71) Applicant: LOCI Orthopaedics Limited, H91 H2Y0 Galway (IE)
(72) Inventor: CLARKE, Gerry, Galway (IE)
(74) Representative: Weldon O'Brien Ltd.

(57) **Abstract**

A broach (1) has a handle and a rod (4), and a detachable head (80, 100) which is configured for fitting in an intramedullary cavity so that it can be used as a trial implant or a permanent implant. The head has an articulating ball socket (87, 107) for connecting to a ball of a proximal implant component for the purposes of a trial during surgery or as a permanent implant. The fastener to attach the broach head to the rod is a threaded socket (86, 106) for threaded engagement with the distal end of the rod. The broach head may have features (120) which do not extend radially as much as the broaching teeth (103) to aid fixation when used as a permanent implant. In another example the head is a trial stem without broaching teeth.

## Description

### Introduction

The present invention relates to intramedullary implants and particularly to surgery for such implants.

It is known to provide broaches with a handle, a rod (or "stem"), and a broach head for the surgeon to create the required space in a bone such as the metacarpal for insertion of an intramedullary stem. Examples are described for example in our published specification EP3914166.

Orthopaedic arthroplasty procedures can be complex and consume large amounts of theatre time. Typical steps involved in implanting a stem in a bone include broaching the bone to size with a series of broaches, removing the final broach, inserting a trial stem, assessing the joint with a trial head, removing the trial stem and the trial head and then inserting an implant stem and head.

The present invention is directed towards providing for improved versatility, and/or accuracy, and/or reduced time for such implant surgery.

### Summary of the Invention

We describe a broach which has a detachable head, so that it may be used for broach purposes in preparation of a bone for an intramedullary stem implant. The head may then be separated and used as a trial implant stem. The head has a threaded socket to receive a male threaded end of a rod of the broach for attachment/detachment. Once a surgeon progresses through a series of broaches and is satisfied that the broach/bone relationship is secure, and the proximal end of the broach head represents the position for the base of a trial stem the surgeon may detach the handle and the broach head may act as the trial stem. This is facilitated by the head having a socket for a ball joint and the head having the same dimensions as the trial stem. Moreover, the head may function as the actual implant stem. To facilitate the engagement of the head as an implant it may have anchor features between at least some teeth, which features do not protrude radially beyond the broach teeth and so do not adversely affect broach function, however they assist subsequent anchoring.

We also describe an intramedullary trial stem for bone joint surgery, the trial stem comprising an elongate intramedullary stem-shaped body and at its proximal end a part of an implant articulating coupler, and wherein the trial stem is removably engageable by a fastener part with a rod extending from a handle, and
the head is configured for friction fitting in an intramedullary cavity so that it is suitable for use as a trial or permanent intramedullary implant stem after disengagement by the fastener part from the rod.

In some examples, the fastener part comprises a threaded socket.

In some examples, the articulating part is a ball socket. In some examples, the body comprises broaching teeth so that the trial stem forms a broach when fastened to a handle rod.

We also describe an intramedullary cavity broach for bone joint surgery, the broach comprising a handle, a rod extending distally, and a broach head with broaching teeth, wherein:
the broach head comprises at its proximal end a part of an implant articulating coupler,
the broach head is removably engageable with the rod by a fastener, and
the broach head is configured for friction fitting in an intramedullary cavity so that it is suitable for use as a trial stem or permanent intramedullary implant after disengagement by the fastener from the rod.

In some examples, the fastener comprises a proximally facing threaded socket in the head and a threaded distal end of the handle rod.

In some examples, at least one of the handle rod and the head have a visual indicator to indicate the extent of fastener thread engagement.

In some examples, the handle rod and the head each have a visible indicator.

In some examples, the indicators are positioned so that when they are aligned close to full engagement the alignment indicates a particular portion of the last turn remaining.

In some examples, the portion is less than 25% of a turn.

In some examples, the portion is in the range of 10% to 15% of a turn.

In some examples, the articulating coupler part comprises a proximally facing socket for engagement with a ball.

In some examples, the fastener comprises a threaded socket extending distally from the articulating ball socket.

In some examples, the head is configured for use as a metacarpal intramedullary implant.

In some examples, the head comprises radially extending bone fixation or osteo anchoring features between at least some juxtaposed broaching teeth, said features extend radially to a lesser extent than adjacent broaching teeth.

In some examples, the features comprise teeth which are directed in the opposite sense to the broaching teeth.

We also describe a method of use of a broach of any example described heren, the method comprising steps of:
manipulating the broach head to remove material from a bone to form an intramedullary cavity in the bone,
inserting the broach head in the cavity so that it fits with a friction fit, separating the rod from the broach head to leave the broach head in position in the bone cavity.
connecting a proximal implant component to the broach head so that the broach head performs the function of a trial implant or a permanent implant.

### Detailed Description of the Invention

The invention will be more clearly understood from the following description of some embodiments thereof, given by way of example only with reference to the accompanying drawings in which:
Fig. 1 is a top view of a broach of the invention,
Fig., 2 is a side view of the broach from a different angle of rotation of the broach about its axis,
Fig. 3 is an end view of the broach,
Fig. 4 is a perspective view of the broach,
Fig. 5 is an enlarged side view showing the distal end of the rod in more detail,
Fig. 6 is a perspective view of a head of the broach, when detached,
Fig. 7 is an end view of the head from the proximal end,
Fig. 8 is a cross-sectional side view in the direction of the arrows A-A of Fig. 7,
Fig. 9 is a top view of the head,
Fig. 10 is a sectional side view of an alternative head, in this case having additional features for bone fixation in which the head can be used as an implant stem, and
Fig. 11 is a side sectional view of an alternative trial stem of the invention.

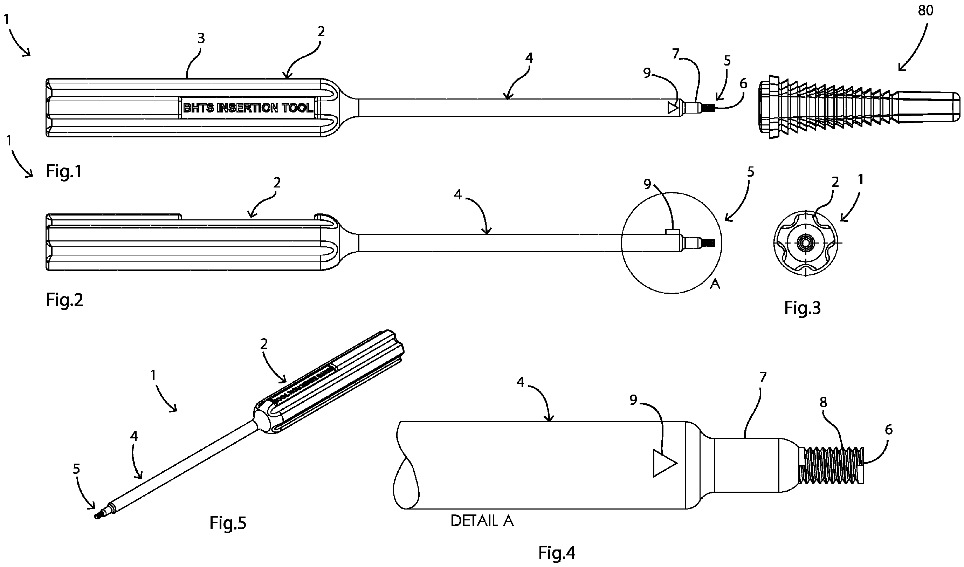

Referring to Figs 1 to 5 a broaching tool 1 comprises a handle 2 with fluted ridges 3 for ease of grip and application of torque. A rod 4 extends distally from the handle and at its distal tip 5 it has a male threaded portion 6 extending from a shoulder 7 of smaller diameter than the rod 4.

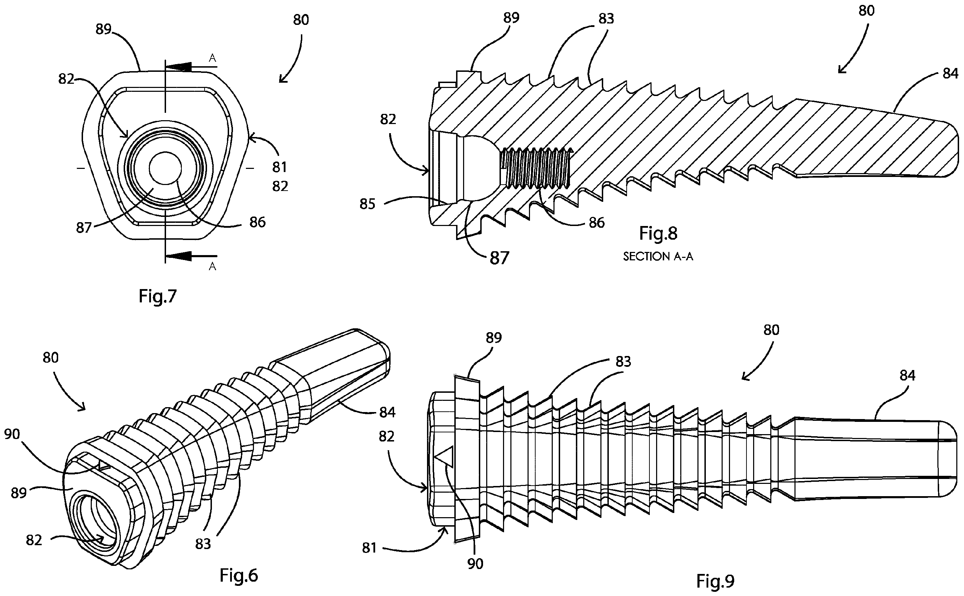

Referring also to Figs. 6 to 9 the head 80 of the tool is shown. The head 80 comprises a unitary main body 81 with a proximal recess 82 around which there is a proximal rim of the body 81. The recess 82 comprises, in order in the distal direction:
a tapered entrance mouth 85,
a ball socket 87, and
a threaded socket 86 matching the male threaded portion 6 of the tool rod.

The head 80 has a series of broach teeth 83 of narrowing diameter in a conventional arrangement for a broach. The head terminates in a non-threaded tip 84.

The rod engages with the head 80 by engagement of the threaded portions 6 and 86. While so engaged, the tool 1 may be used in a conventional manner to create an opening for an intramedullary stem in a bone, in this case the metacarpal. Because of the very tight engagement of the parts 6 and 86 the tool works just as if the head and the rod were integral. The tightness of the threaded connection is optimised by use of visual indicia in the form of arrows in this example, there being a raised arrow 9 at the distal end of the rod 4, just before the shoulder 7, and a corresponding raised arrow 90 on the body 81 of the head. These arrows are positioned, and the threads are ground, so that when the arrows are aligned when there is about 1/8th of a rotation remaining before the threads lock together.

The arrow 90 is shown as being in the plane of the surface, however in other examples the indicator may be raised in three dimensions.

In general, the indicators are positioned so that when they are aligned the alignment indicates a particular portion of the last turn remaining. The portion is preferably less than 25%., and more preferably is in the range of 10% to 15% of a turn.

Fig. 1 shows the full broach, including the head 80. After the tool 1 has been used for its broach function the surgeon can decouple the rod from the broach head and the broach head may then be used as a trial stem as it's already in the cavity to check along with trial heads for the degree of distraction within the joint. The handle may then be reattached, the broach head 80 withdrawn and the relevant implant stem may then be inserted into the cavity. By using the head as a trial stem, the number of tool components is reduced. The fact that the broach head has the ball socket 87 facilitates its use as a trial stem.

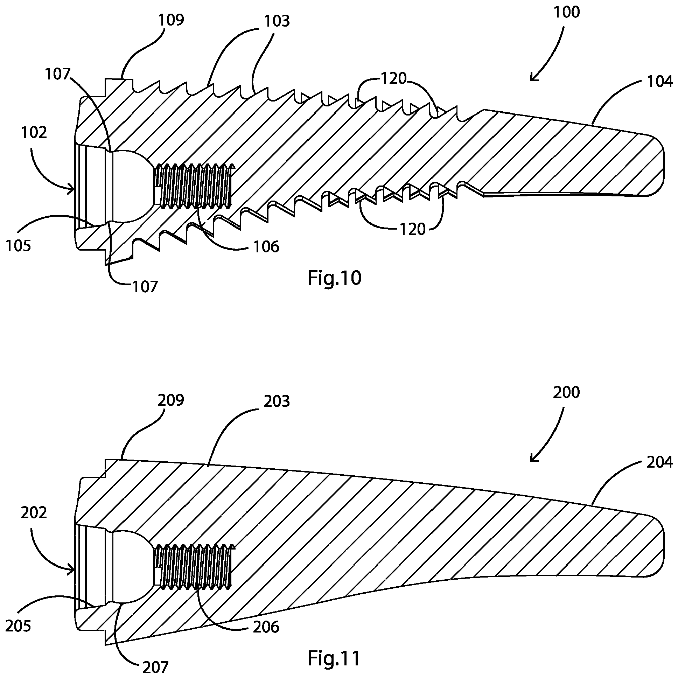

In another example the broach head may be used as the actual implant stem. In this case it is envisaged that there may be features between the broach teeth to facilitate integration. Referring to Fig. 10 a broach head 100 has a proximal rim 109 surrounding a recess with a mouth 105, a ball socket 107, and a threaded socket 106. There are broaching teeth 103 and a distal non-toothed tip 104. There are also anchoring features 120 between some of the broaching teeth 103.

It will be appreciated that invention provides multifunctionality for a broaching tool used in the implantation of a stem into a bone. By permitting the handle to be detachable from the head and by constructing the internal geometry of the head such that it contains an articulating socket, the broach head may be used as a trial stem and may articulate with a trial head possessing a ball which mates with the socket 87/107 within the broach head/trial stem. Furthermore, the broach head may act as the actual implant stem.

Orthopaedic arthroplasty procedures can be complex and consume large amounts of theatre time. Typical steps involved in implanting a stem in a bone include broaching the bone to size with a series of broaches, removing the final broach, inserting a trial stem, assessing the joint with a trial head, removing the trial stem and the trial head and then inserting an implant stem. This invention eliminates the step of inserting a trial stem. The further embodiment eliminates the final step of inserting the implant stem, by having the broach head act as both the trial stem and the implant stem.

In some examples, the broach heads are sized to be the same as every size stem used in a range of stem sizes. The indications 9 and 89 are a particularly simple and effective means of indicating that 1/8th of a turn remains before the components are locked together. In this manner, the tool may function as a broach, but the handle may readily be disengaged from the broach head.

Thus, the handle may be conveniently detached from the broach head and the broach head may remain temporarily in the bone to function as a trial stem and interact with a trial head, thereby facilitating a surgeon to evaluate the joint for appropriate implant sizing. This interaction is facilitated by the hemispherical socket 87/107 which permits a ball to be inserted therein and function as a ball and socket joint with ball capture at the annular snap-fit 107.

In some examples the trial head may be removed and then the handle may be re-attached to the broach head so that the broach head may be withdrawn and an implant stem may be inserted in the bone.

In a further embodiment, the broach head may be left *in situ* and may itself function as an implant stem. To facilitate secure anchorage in the bone, the proximal side of a number of cutting teeth may incorporate osteo anchoring, such as described in US8,888,862 or other fixation features, although these are not essential.

Materials chosen must be such that a bearing surface comprises one side of the ball and socket joint. The broach head whether for use as a trial stem or as an implant, preferably comprises a metal such as Titanium, Cobalt-Chrome alloy or stainless-steel alloys and the mating ball head made from an engineering polymer with good biocompatibility such as PEEK or carbon-reinforced PEEK. Alternatively, the broach may be made from PEEK or carbon-reinforced PEEK and the head made from Titanium, Cobalt-Chrome alloy or stainless-steel alloys.

Referring to Fig. 11 a trial stem 200 of another embodiment is illustrated. The trial stem is configured to fit to a handle and a handle rod but does not have broach teeth. It comprises an integral body 203 of PEEK material with a proximal shoulder around an opening having in order from the proximal end distally:
a mouth 205,
an articulating ball socket 207 with a snap-fit entrance, and
a threaded socket 206 for engagement with a rod extending from a handle such as the handle 2 and rod 4.

In this case the trial stem 200 is not part of a broach but may be used with the handle and other parts of the broach as described above. It is configured to match the shape of the implant stem, and the threaded socket 206 allows it to be placed in position during surgery to check the extent of broaching of the bone. The ball socket 207 allows a trial implant proximal component to be engaged with it by snap fitting of its head into the socket 207 This then permits the surgeon to evaluate the laxity or otherwise of the joint space and decide which size of proximal component implant is appropriate.

Components of embodiments can be employed in other embodiments in a manner as would be understood by a person of ordinary skill in the art. The invention is not limited to the embodiments described but may be varied in construction and detail.

## Claims

1. An intramedullary trial stem (80, 100, 200) for bone joint surgery, the trial stem comprising an elongate intramedullary stem-shaped body and at its proximal end a part (87, 107, 207) of an implant articulating coupler, and wherein the trial stem is removably engageable by a fastener part (86, 106, 206) with a rod (4) extending from a handle (2), and
the head is configured for friction fitting in an intramedullary cavity so that it is suitable for use as a trial or permanent intramedullary implant stem after disengagement by the fastener part (86, 106, 206) from the rod.

2. An intramedullary trial stem (80, 100, 200) as claimed in claim 1, wherein the fastener part comprises a threaded socket (86, 106, 206).

3. An intramedullary trial stem (80, 100, 200) as claimed in claim 1 or claim 2, wherein the articulating part is a ball socket (87, 107, 207).

4. An intramedullary trial stem (80, 100, 200) as claimed in any preceding claim, wherein the body comprises broaching teeth (83, 103) so that the trial stem forms a broach when fastened to a handle rod (4).

5. An intramedullary cavity broach (1) for bone joint surgery, the broach comprising a handle (2), a rod (4) extending distally, and a broach head (80, 100) with broaching teeth (83, 103), wherein:
the broach head comprises at its proximal end a part (87, 107) of an implant articulating coupler,
the broach head is removably engageable with the rod by a fastener (86, 106), and
the broach head is configured for friction fitting in an intramedullary cavity so that it is suitable for use as a trial stem or permanent intramedullary implant after disengagement by the fastener from the rod.

6. An intramedullary cavity broach as claimed in claim 5, wherein the fastener comprises a proximally facing threaded socket (86, 106) in the head and a threaded distal end (5) of the handle rod (4)

7. An intramedullary cavity broach as claimed in claim 6, wherein at least one of the handle rod and the head have a visual indicator (9, 90) to indicate the extent of fastener thread engagement.

8. An intramedullary cavity broach as claimed in claim 7, wherein the handle rod and the head each have a visible indicator (9, 90).

9. An intramedullary cavity broach as claimed in claim 8, wherein the indicators are positioned so that when they are aligned close to full engagement the alignment indicates a particular portion of the last turn remaining.

10. An intramedullary cavity broach as claimed in claim 9, wherein the portion is less than 25% of a turn.

11. An intramedullary cavity broach as claimed in claim 10, wherein the portion is in the range of 10% to 15% of a turn.

12. An intramedullary cavity broach as claimed in any preceding claim, wherein the articulating coupler part comprises a proximally facing socket (87, 107) for engagement with a ball.

13. An intramedullary cavity broach as claimed in claim 12, wherein the fastener comprises a threaded socket (86, 106) extending distally from the articulating ball socket (87, 107).

14. An intramedullary cavity broach as claimed in claim 13, wherein the head is configured for use as a metacarpal intramedullary implant.

15. An intramedullary cavity broach as claimed in any of claims 5 to 14, wherein the head comprises radially extending bone fixation or osteo anchoring features (120) between at least some juxtaposed broaching teeth (103), said features extend radially to a lesser extent than adjacent broaching teeth, and optionally the features comprise teeth (120) which are directed in the opposite sense to the broaching teeth.
